Europäisches Patentamt

European Patent Office

Office européen des brevets

(11) Publication number: 0 085 254 A1

# EUROPEAN PATENT APPLICATION

(21) Application number: 82306895.2

(22) Date of filing: 23.12.82

(51) Int. Cl.³: A 61 K 35/14
A 61 K 37/02, C 07 G 7/00

(30) Priority: 28.12.81 JP 211096/81
28.12.81 JP 211097/81

(43) Date of publication of application:
10.08.83 Bulletin 83/32

(84) Designated Contracting States:
CH DE FR GB LI SE

(71) Applicant: Sakagami, Yoshio
16-19-603 Jiyugaoka 3-chome
Meguro-ku Tokyo(JP)

(72) Inventor: Sakagami, Yoshio
16-19-603, Jiyugaoka 3-chome
Meguro-ku Tokyo(JP)

(72) Inventor: Manabe, Hofumi
No. 5-27, Inogashira 2-chome
Mitaka-shi Tokyo(JP)

(74) Representative: Perry, Robert Edward et al,
GILL JENNINGS & EVERY 53-64 Chancery Lane
London WC2A 1HN(GB)

(54) Proteins and their extraction.

(57) Proteins designated Santacuarators have been extracted from animal blood, serum or organs, and which can regulate cancer cell growth. Three Santacuarators have been identified and characterised, as have coextracted antagonistic substances.

EP 0 085 254 A1

## PROTEINS AND THEIR EXTRACTION

The present invention relates to proteins which can be extracted from human or animal blood, serum or organs, and complexes and extracts containing them.

Current developments in the field of anti-tumour research have involved studies in areas such as lympho-kines (see references 1,2,3), tumour necrosis factor (TNF ) (4,5,6,7,8,9,10) and  humoral antibody (11).  It has been proposed to use them by conventional methods in the treatment of cancer, by killing malignant tumours. Accordingly, they have also exerted an undeniable influence on normal cells.  In recent years, furthermore, there have been reported some reversals of malignant tumour cells, i.e. these cells have lived and reverted to normal cells (see references 12,13,14,15,16).  The references are:

1      Bagshawe, K.D., and Currie, G.A. Nature 218, 1253 - 1254 (1968)

Mani, R.N., Bryceson, A.D.M., Wolstencroft, R.A., and Dumonde, D.C. Nature 224, 43 - 44 (1969)

Dumonde, D.C., Wolstencroft, R.A., Panayi, G.S., Matthew, M., Morley, J., and Howson, W.T. Nature 224, 38 - 42 (1969)

Carswell, E.A., Old, L.J., Kassel, R.L., Green, S., Fiere, N., and Williamson, B. Proc. natn. Acad. Sci. U.S.A. 72, 3666 - 3670 (1975)

Matthews, N., and Watkins, J.F. Br. J. Cancer 38, 302 - 309 (1978)

Matthews, N. Br. J. Cancer 38, 310 - 315 (1978)

Ostrove, J.M., and Gifford, G.E. Proc. Soc. Exp. Biol. Med. 160, 354 - 358 (1979)

Helson, L., Helson, C., and Green, S. Exp. Cell Biol. 47, 53 - 60 (1979)

Clark, I.A. Infect Immun. 24, 319 - 325 (1979)

Haskill, S., Ritter, F., and Becker, S. J. Immunol. 125, 454 - 458 (1980)

Lando, P., Blomberg, F., Berzins, K., and Perlmann, P. Cancer Res. 40, 1691 - 1698 (1980)

Blanock, J.E., and Gifford, G.E. Proc. natn. Acad. Sci. U.S.A., 74, 5382 - 5386 (1977)

Dion, L.D., Blalock, J.E., and Gifford, G.E. Exp. Cell Res. 117, 15 - 22 (1978)

Honma, Y., Kasukabe, T., and Hozumi, M. Cancer Res. 39, 2190 - 2194 (1979)

Honma, Y., Kasukabe, T., Okabe, J., and Hozumi, M. Gann 68, 241 - 246 (1977)

Kasukabe, T., Honma, T., and Hozumi, M. Gann 70, 119 - 123 (1979)

3

It has now been found that there are proteins, present at very low levels, in animal, especially mammalian, e.g. human, blood, serum, organs and the like, which can exhibit, in vitro, a regulative action on proliferation of cancer cells and, in vivo, a life-saving effect on cancerous animals. These proteins have been named Santacuarators A, B and C. These "Santacuarators" can have therapeutic utility owing to their regulative action on the proliferation of cancer cells, irrespective of whether they are in refined form or in the form of a crude powder or mixture obtained at a suitable stage in the manufacturing process.

It has also been found that blood, serum or organs include, in addition to Santacuarators, a substance antagonistic thereto.

This substance is antagonistic in vitro to the Santacuarator' regulative action on proliferation of cancer cells and exerts in vivo a short life effect on cancer cell burdened animal, and its physiological activity is totally contrary to that of Santacuarator. The present inventors named this substance Daemocuarator because of its chemical, physical and biological characteristics.

In other words, one embodiment of the present invention consists in blood, serum, extract of removal organ or the like having an improved regulative action on proliferation of cancer cells that is obtained by subjecting an aqueous extract of human or animal blood, serum or removal organ to extractive treatment using an organic solvent or treatment using an adsorbent or concurrent use of both treatments and thus removing or reducing Daemocuarator, namely a substance antagonistic to Santacuarator (the factor having a regulative action on proliferation of cancer cells).

Another embodiment of the present invention consists in a glycoprotein named Santacuarator A fractionated from human or animal blood, serum or extract of removal organ and having a regulative action on proliferation of cancer cells, which is a white powder having a melting point of 240 - 250°C (decomposition); has a molecular weight of about 74000 measured by 10% sodium dodecylsulfate electrophoresis; is soluble in water and insoluble in butanol, acetone, ethyl acetate, chloroform, benzene and hexane; has an aminoacid composition comprising aspartic acid 9.7%, threonine 5.5%, serine 12.3%, glutamic acid 10.0%, proline 5.8%, glycine 7.7%, alanine 7.5%, valine 8.8%, methionine 0.8%, isoleucine 3.2%, leucine 7.5%, tyrosine 3.5%, phenylalanine 3.5%, lysine 6.2%, histidine 1.8% and arginine 4.0%; is positive in all

In other words, by changing the saturation degree of salt for instance into 40%, 90% and 100% in the practice of abovementioned salting out, fractional precipitating in accordance with varied molecular weights, enhancing the purity of precipitates by molecular sieve adsorption and elusion and thereafter carefully fractionating said precipitates there can be obtained the following Santacuarator A, B and C.

Santacuarator A

| | | |
|---|---|---|
| External appearance | : | white powder |
| Melting point | : | 240 - 250°C (decomposition) |
| Property | : | glycoprotein |
| Molecular weight | : | about 74000 (determined by 10% sodium dodecylsulfate electrophoresis) |
| Solubility | : | soluble in water and insoluble in butanol, acetone, ethyl acetate, chloroform, benzene and hexane |
| Aminoacid composition | : | aspartic acid 9.7%, threonine 9.3%, serine 12.3%, glutamic acid 10.0%, proline 5.8%, glycine 7.7%, alanine 5.8%, valine 8.8%, methionine 0.8%, isoleucine 3.2%, leucine 7.5%, tyrosine 3.5%, phenylalanine 3.5%, lysine 6.2%, histidine 1.8% and arginine 4.0% |
| Color reaction | : | positive in ninhydrine, anthrone, Molish and periodic acid reactions |
| Infrared absorption spectrum | : | refer to Fig. 1 [measured with KBr pellet] |
| Ultraviolet absorption spectrum | : | refer to Figs. 2, 3 and 4 absorption maximum 279 nm in pH 3 water (refer to Fig. 2) |

0085254

invention can be obtained from human or animal blood, serum or extract of removal organ by the use of proper combinations of known operations such as salting out, extraction, absorption, elution, dialysis, fractionation, precipitation, filtration, precipitation on isoelectric point, utilization of ion-exchange resin, electrophoresis and the like depending on the property of material used.

More preferably, crude powders of Santacuarator can be obtained in the manner of first adding ammonium sulfate, sodium chloride (common salt) or the like to mammalian blood, serum or aqueous extract of removal organ such as liver, kidney or the like, thereby salting out the effective component, dialyzing said precipitate and then drying.

The thus obtained crude powders may be further refined by using, singly or in combination, column chromatography charged with ion-exchange resin Sephadex, active carbon, silica gel, diethylaminoethyl (DEAE) cellulose or the like, electrophoresis using polyacrylamide gel, saccharose or the like, precipitation on isoelectric point or the like, and besides Santacuarator may be refined by making an adsorbent such as silica gel, bentonite, acid clay, active carbon or the like adsorb it thereon.

The protein substance Santacuarator thus separated from human or animal blood, serum or extract of removal organ exhibits an extremely specific physiological activity as described above and so is very useful as an antitumor agent. The present inventors have continued their studies on this novel protein Santacuarator to find that said protein is not a single substance but may be separated into some components which have each a regulative action on proliferation of cancer cells. These components were named Santacuarator A, B and C.

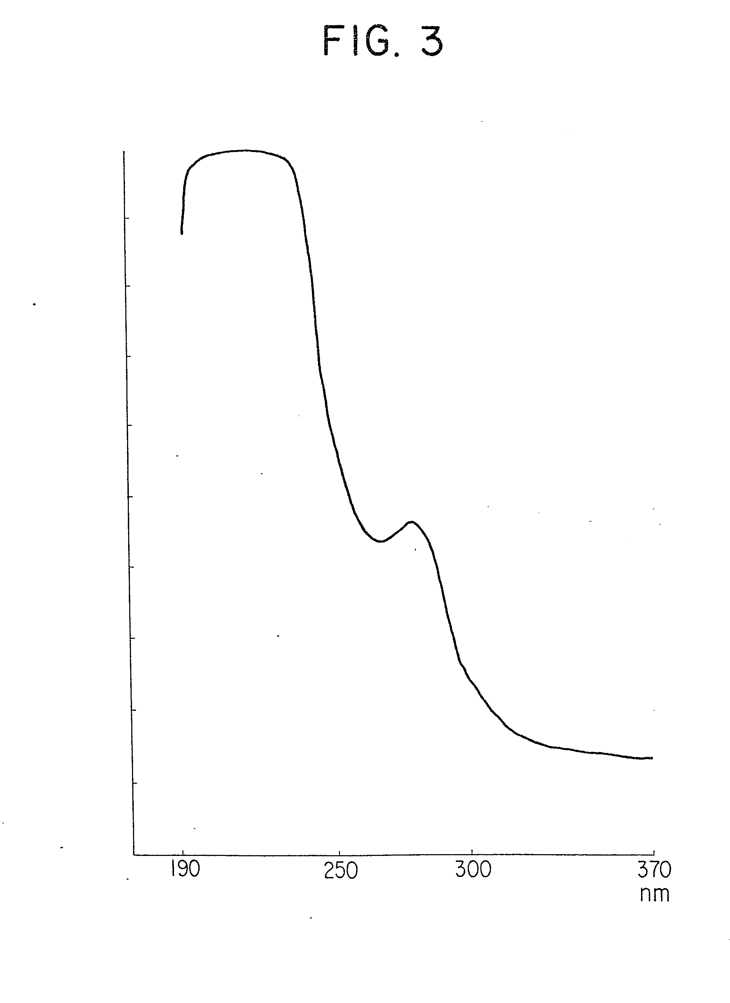

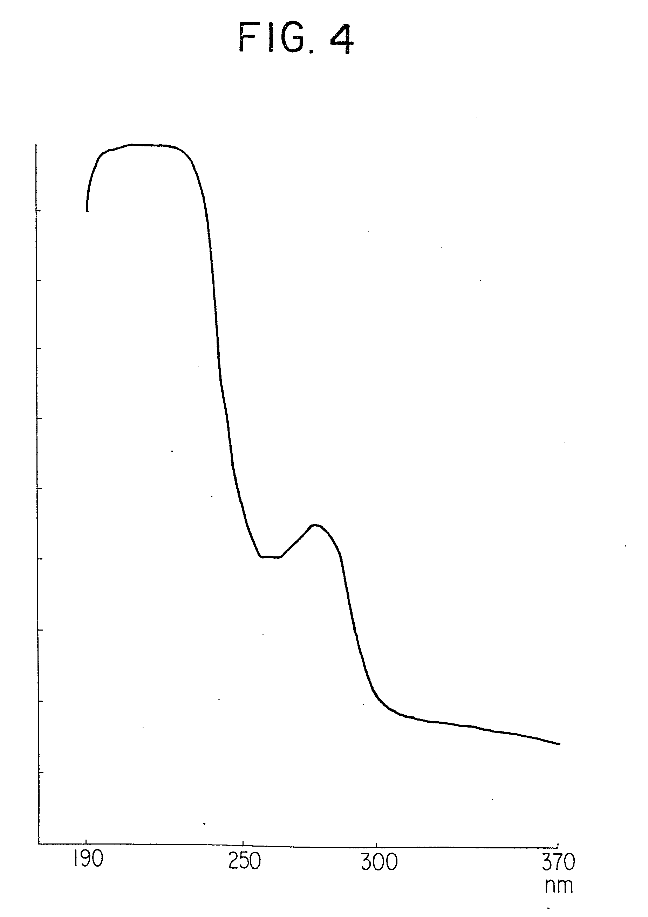

absorption maximum 278 nm in pH 7 water (refer to Fig. 3) absorption maximum 277 nm in pH 10.0 water (refer to Fig. 4)

Santacuarator B

External appearance : white powder

Melting point : 220 – 225°C (decomposition)

Property : Protein

Molecular weight : about 22000 (determined by 10% sodium dodecylsulfate electrophoresis)

Solubility : soluble in water and insoluble in acetone, ethyl acetate, benzene, hexane and chloroform

Aminoacid composition: aspartic acid 8.9%, threonine 6.6%, serine 6.4%, gultamine acid 10.9%, proline 4.8%, glycine 4.4%, alanine 7.1%, cysteine 12.3%, valine 5.8%, methionine 0.7%, isoleucine 2.4%, leucine 8.8%, tyrosine 2.8%, phenylalanine 5.0%, lysine 7.6%, histidine 2.5% and arginine 3.6%

Color reaction : positive in ninhydrine reaction and negative in anthrone, Molish and periodic acid reactions

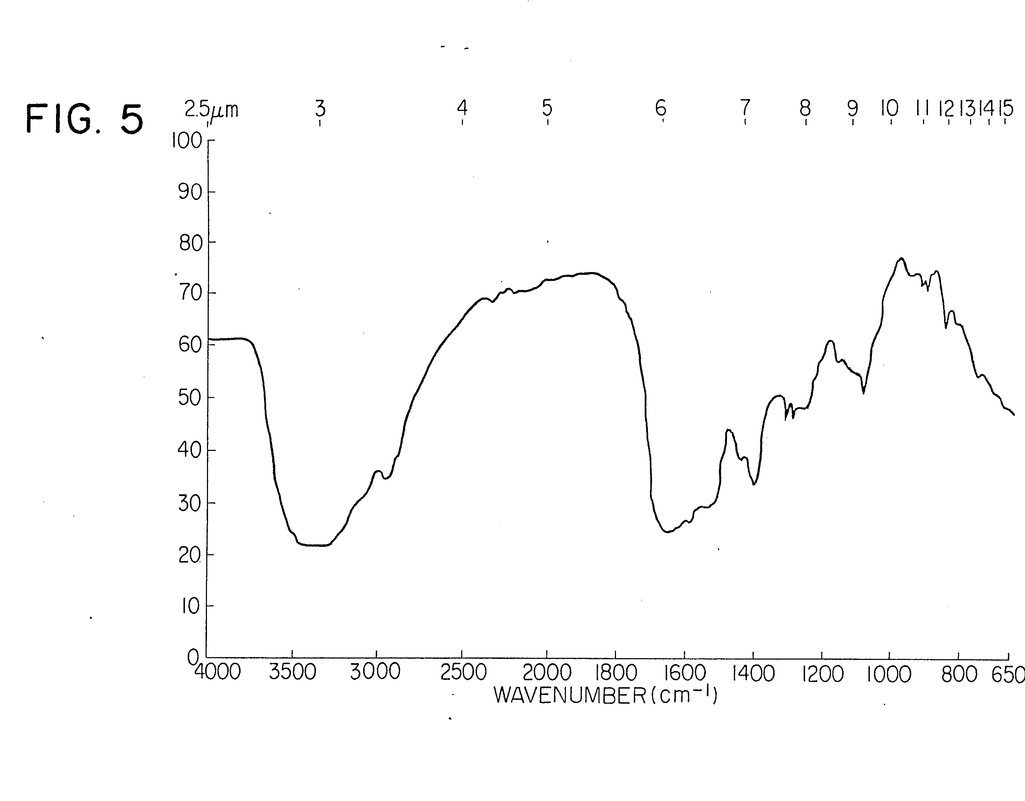

Infrared absorption spectrum : refer to Fig. 5 [measured with KBr pellet]

Ultraviolet absorption spectrum :

absorption maximums 283 nm and 289 nm in pH 3 water (refer to Fig. 6) absorption maximum 280 nm in pH 7 water (refer to Fig. 7) absorption maximum 278 nm in pH 10.0 water (refer to Fig. 8)

Santacuarator C

External appearance : white powder

Melting point : 235 - 240°C (decomposition)

Property : protein

Molecular weight : about 14000 (determined by 10% sodium dodecylsulfate electrophoresis)

Solubility : soluble in water and insoluble in acetone, ethyl acetate, benzene, hexane and chloroform

Aminoacid composition : aspartic acid 10.5%, threonine 7.1% serine 6.2%, gultamic acid 12.5%, proline 5.2%, glycine 4.7%, alanine 8.0%, cysteine 1.7%, valine 6.1%, methionine 0.7%, isoleucine 2.7%, leucine 10.5%, tyrosine 3.1%, phenylalanine 5.7%, lysine 8.5%, histidine 2.8% and arginine 4.1%

Color reaction : positive in ninhydrine reaction and negative in anthrone, Molish and periodic acid reactions

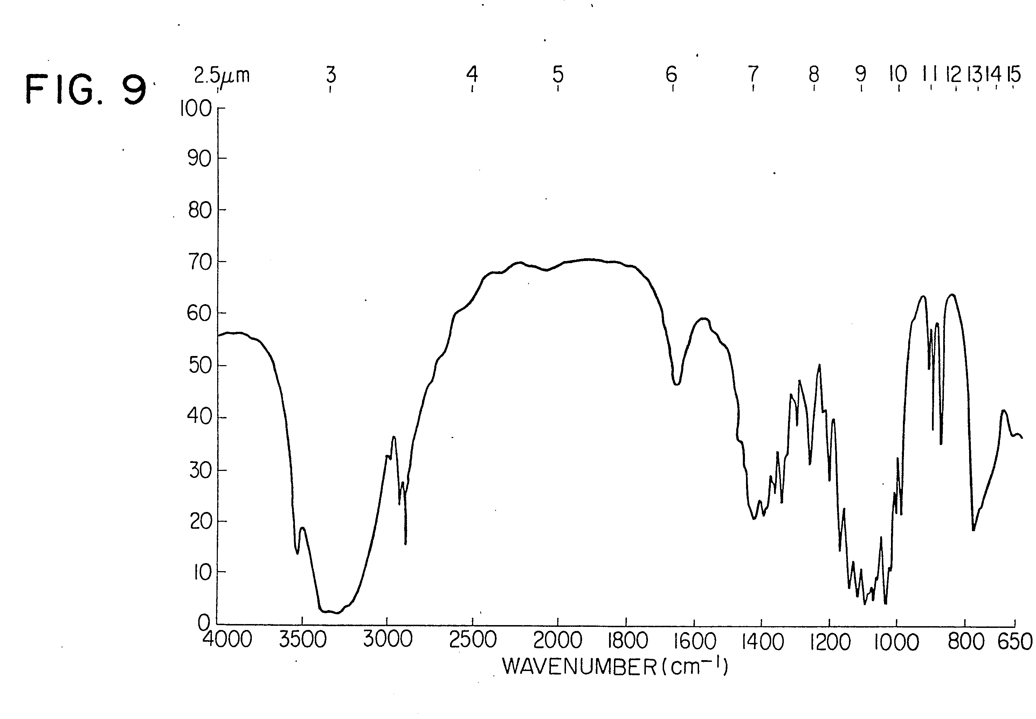

Infrared absorption spectrum : refer to Fig. 9 [measured with KBr pellet]

Ultraviolet absorption spectrum :

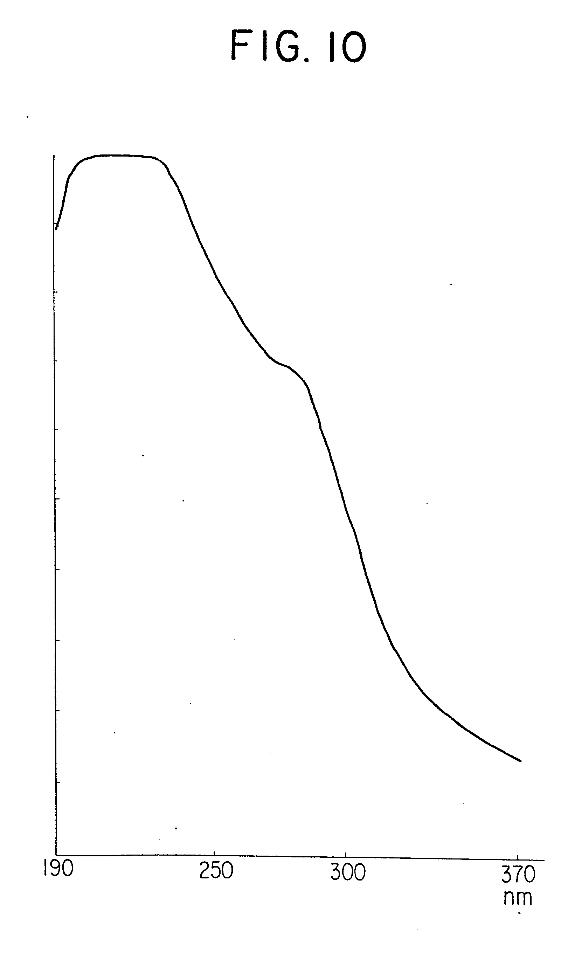

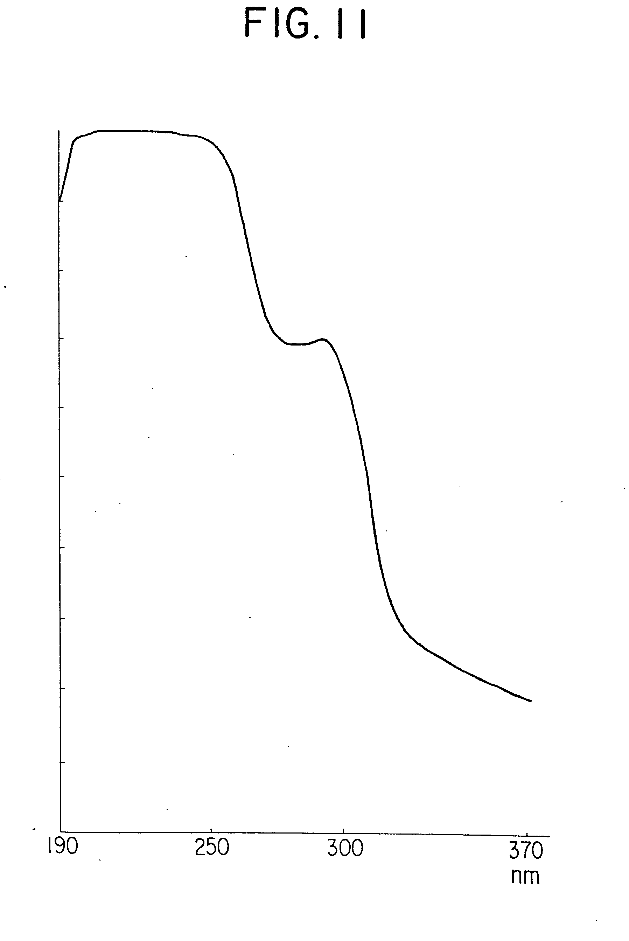

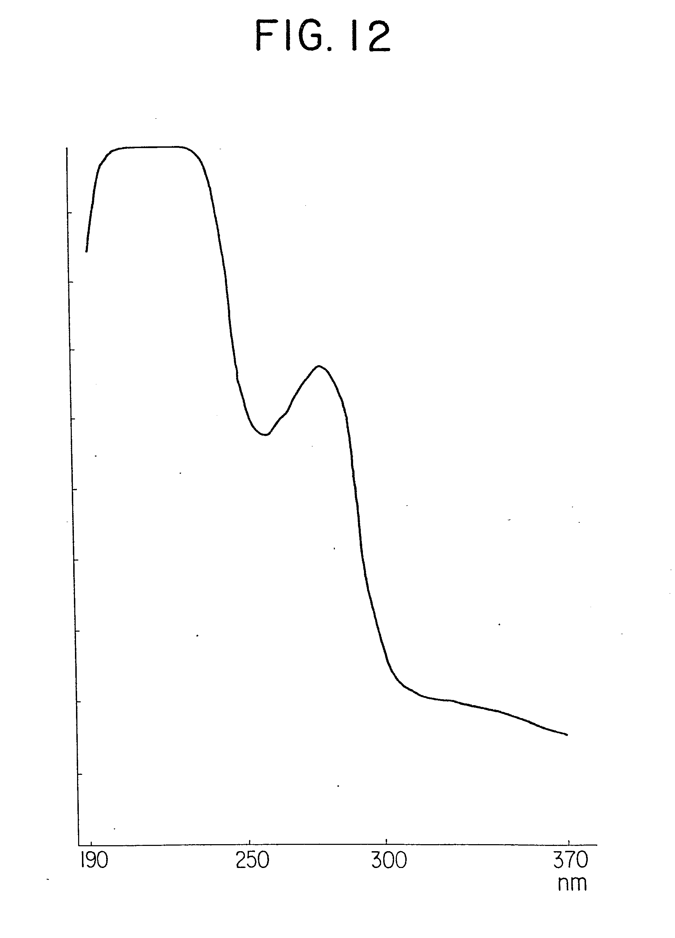

shoulder 280 nm in pH 3 water (refer to Fig. 10) absorption maximum 290 nm in pH 7 water (refer to Fig. 11) absorption maximum 277 nm in pH 10.0 water (refer to Fig. 12)

According to 7% acrylamide gel electrophoresis, the above mentioned Santacuarator A, Santacuarator B and Santacuarator C do not transfer from their original points at pH 6.5, and transfer therefrom at pH 8.0, pH 9.5 and pH 10.0 respectively to exhibit a single band.

Accordingly, fractionation can also be made by acrylamide gel electrophoresis in lieu of chromatography. It is needless to say that Santacuarator A, Santacuarator B and Santacuarator C obtained according the aforesaid refining process can be used as agents for regulating proliferation of cancer cells. In addition, their crude powder obtained at respective stages of the refining process can also be used for various purposes as agents for regulating proliferation of cancer cells. This Santacuarator is effective in the simple form free of or in the mixture form with other components, and can be prepared into various medicine forms e.g. for injection. Santacuarator should be said to have the specific physiological activity in that it has a regulative action on proliferation of cancer cells and exhibits a marked life saving effect on cancer cell burdened animal, especially in that it has a strong regulative action on proliferation of cancer cells, while it does not exhibit in vitro any killing action on cancer cells, is of no virulence against human beings and animals and does not exert any notable regulative action on normal cells, and therefore should be extremely valuable as medicine.

As is evident from the aforegoing, Santacuarator is surely possessed of the specific physiological activity but on the other hand it is in need of the comparatively complicated separation-refining process.  In view of this, one embodiment of the present invention is directed toward improvements in the regulative action on proliferation of cancer cells inherent in Santacuarator, wherein Santacuarator is not isolated from but Daemocuarator is removed from blood, serum or extract of organ or the like, said Daemocuarator being a substance contained in the extract together with Santacuarator and antagonistic thereto.

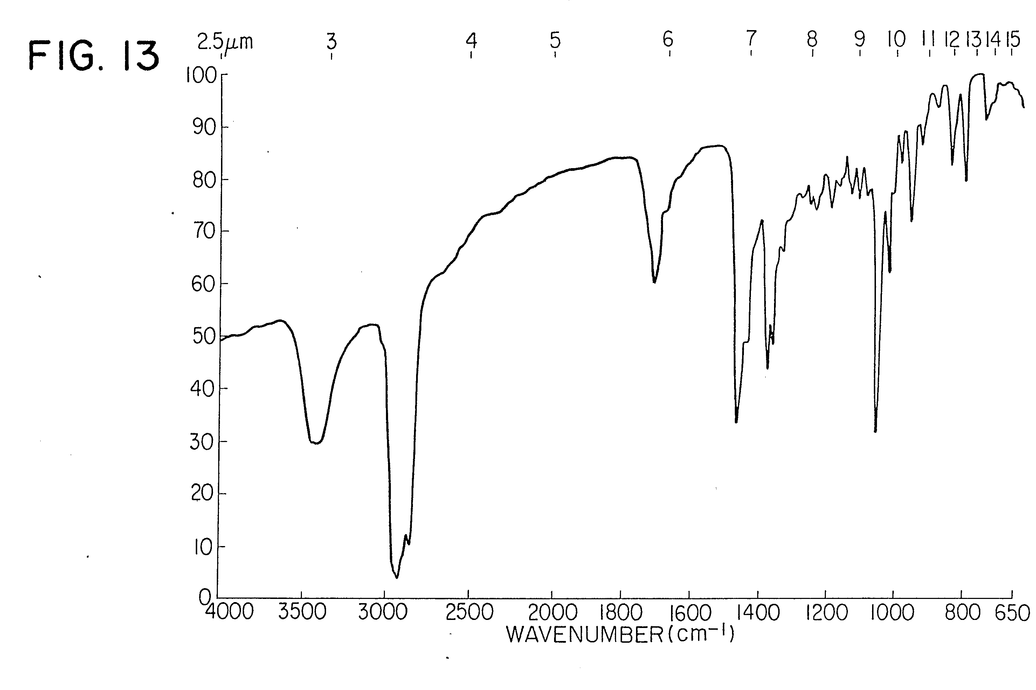

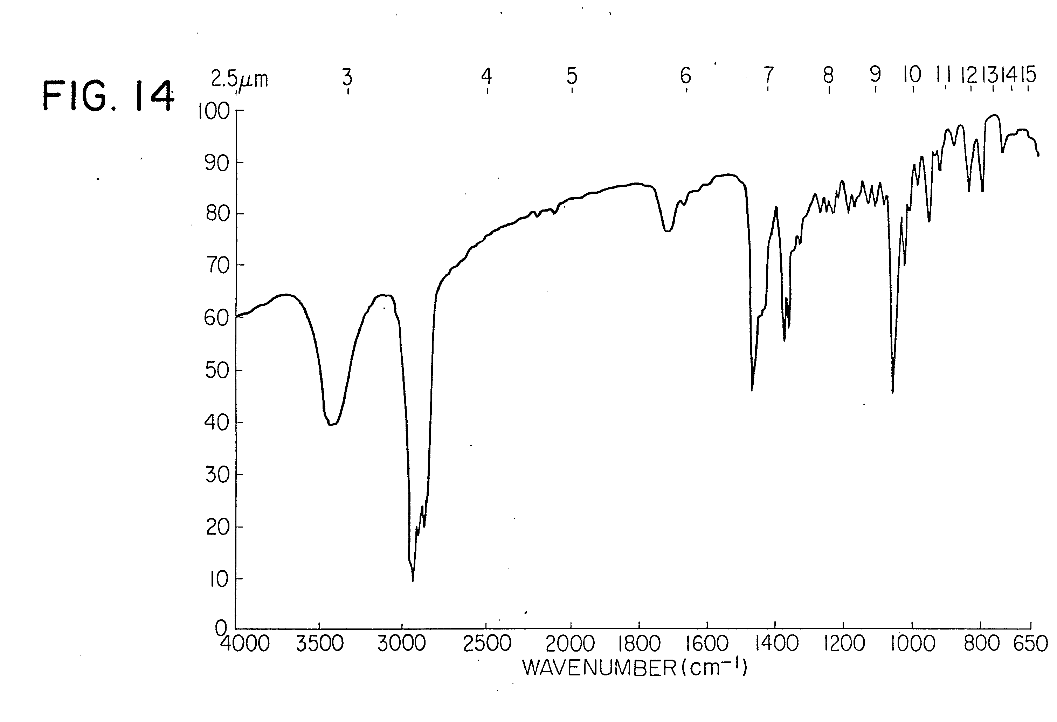

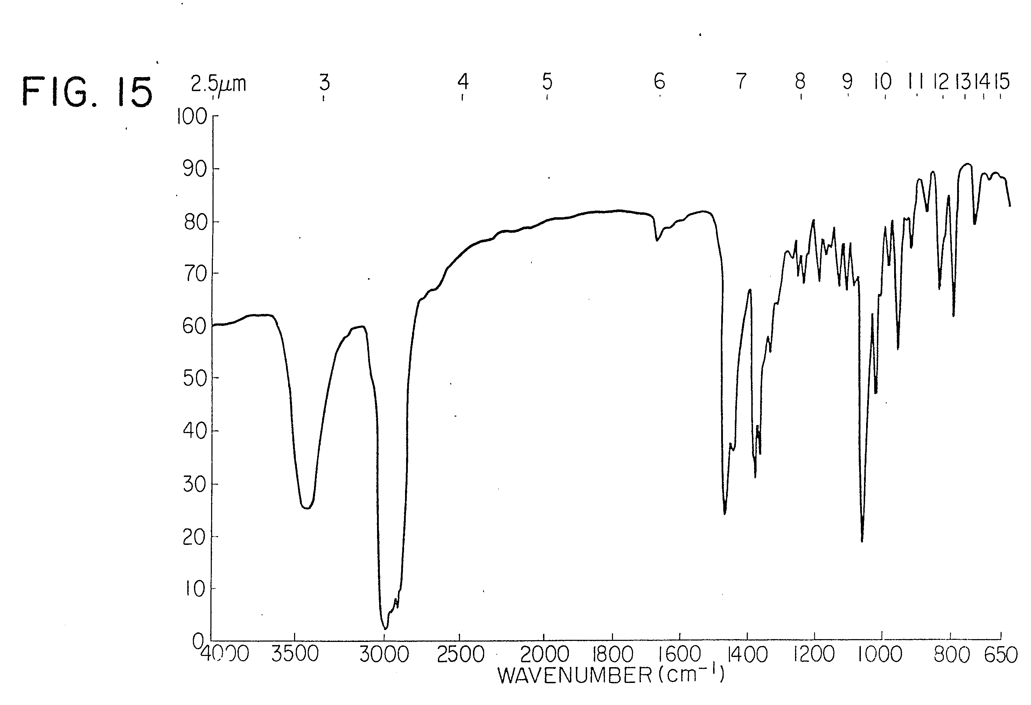

This Daemocuarator is a colorless needle crystalline neutral substance, is soluble in organic solvents, and seems to be not a simple substance but comprised of Daemocuarator A, B, C and so on (refer to        Figures     13 - 15).

These components A, B and C are common in that they are water-insoluble, are soluble in organic solvents and exhibit, both in vitro and in vivo, an antagonistic action to the Santacuarator group.  Due to this, these Daemocuarator A, B and C will be named Daemocuarator generically in the present invention.

As described above, Santacuarator is water-soluble and insoluble in organic solvents, while Daemocuarator is water-insoluble and soluble in organic solvents.  Therefore, Daemocuarator may be said to be antagonistic in physiological activity to Santacuarator.  Accordingly, the removal of this Daemocuarator from blood or extract of organ permits to enhance the physiological activity of Santacuarator and obtain blood, serum or extract of removal organ effective for the purpose of regulating proliferation of cancer cells even when dispensing with the step of separation-refining Santacuarator.

Separation of Daemocuarator, the substance antagonistic to Santacuarator, from blood, serum or extract of removal organ may blood, serum or extract of organ by means of adsorption method because Daemocuarator has a property of being adsorbed readily on the adsorbent such as diatomaceous earth, clay, kaolin, silica gel, alumina, active carbon or the like.

The Daemocuarator-removed or-reduced blood, serum or extract of organ according to the present invention may be used, as it stands, for the purpose of medical treatment because it is able to effectively regulate the proliferation of cancer cells without killing them, can display the inherent physiological activity in Santacuarator, namely the animal life saving effect, to the full, and has an improved regulative action on proliferation of cancer cells, besides may be prepared into various medicine shapes by combination with other medicines. The precipitate obtained by the addition of a salt such as ammonium sulfate or an organic solvent such as alcohol or the like to this extract, namely Santacuarator complex, is also possessed of the inhibitated action on proliferation of cancer cells, and further the dried and aqueous Santacuarator complexes are also available for the purpose of inhibiting the proliferation of cancer cells. The blood, serum or extract of organ according to the present invention may be said to have a point of advantage worthy of special mention as compared with usual medicines used for regulating proliferation of cancer cells in that even the extract obtained from treatment of a living body component such as blood or the like does not exhibit any virulence on human bodies.

Next, the present invention will be more detailed with reference to embodiments, wherein, however, the kinds of additives and their mixing ratios should not be limited to those shown in the respective embodiments and may be varied in wide ranges.

0085254

be achieved by subjecting blood, serum or aqueous extract of organ to direct extraction treatment using an organic solvent or adsorption treatment using an adsorbent or the combination of both treatments making use of the abovementioned characteristics of Daemocuarator being soluble in an organic solvent and easily adsorbed on an adsorbent, thereby removing Daemocuarator therefrom.

As the organic solvent there may be used aromatic and aliphatic hydrocarbons, chlorinated hydrocarbons, alcohols, ketones, esters, ethers and nitriles, and as the adsorbent there may be used any optional one of active carbon, silica gel, diatomaceous earth, clay, kaolin, alumina and the like, especially preferably utilized benzene, ethyl acetate, chloroform, hexane, butanol, methylethyl ketone and the like. In this connection, it is to be noted that in the case of blood or serum it is possible to use acetone, alcohol and the like, but in the case of an aqueous extract of organ it is desirable to selectively use an organic solvent or an adsorbent that has no miscibility with water.

As the result of the abovementioned simple operation of extracting blood, serum or extract of organ with an organic solvent or adsorbing it using an adsorbent, Santacuarator, a factor acting to regulate proliferation of cancer cells, is allowed to remain in the water phase, while Daemocuarator, a substance antagonistic to Santacuarator, transfers to the organic phase or adsorption phase, whereby both are separated from each other and blood, serum or extract of organ improved in the regulative action on proliferation of cancer cells can be obtained, that is the action of Santacuarator.

Daemocuarator may be removed or reduced easily from

0085254

in acetone, ethyl acetate, benzene, hexane and chloroform; has
an aminoacid composition comprising aspartic acid 10.5%, threonine
7.1%, serine 6.2%, gultamic acid 12.5%. proline 5.2%, glycine
4.7%, alanine 8.0%, cysteine 1.7%, valine 6.1%, methionine 0.7%,
isoleucine 2.7%, leucine 10.5%, tyrosine 3.1%, phenylalanine 5.7%,
lysine 8.5%, histidine 2.8% and arginine 4.1%; is positive in
ninhydrine reaction and negative in anthrone, Molish and periodic
acid reactions; represents the infrared absorption spectrum [KBr
pellet] shown in Fig. 9; and exhibits the shoulder of ultraviolet
absorption at 280 nm in pH 3 water and the maximum of ultraviolet
absorption at 290 nm in pH 7 water and at 277 nm in pH 10.0 water.

Brief Description of The Drawings

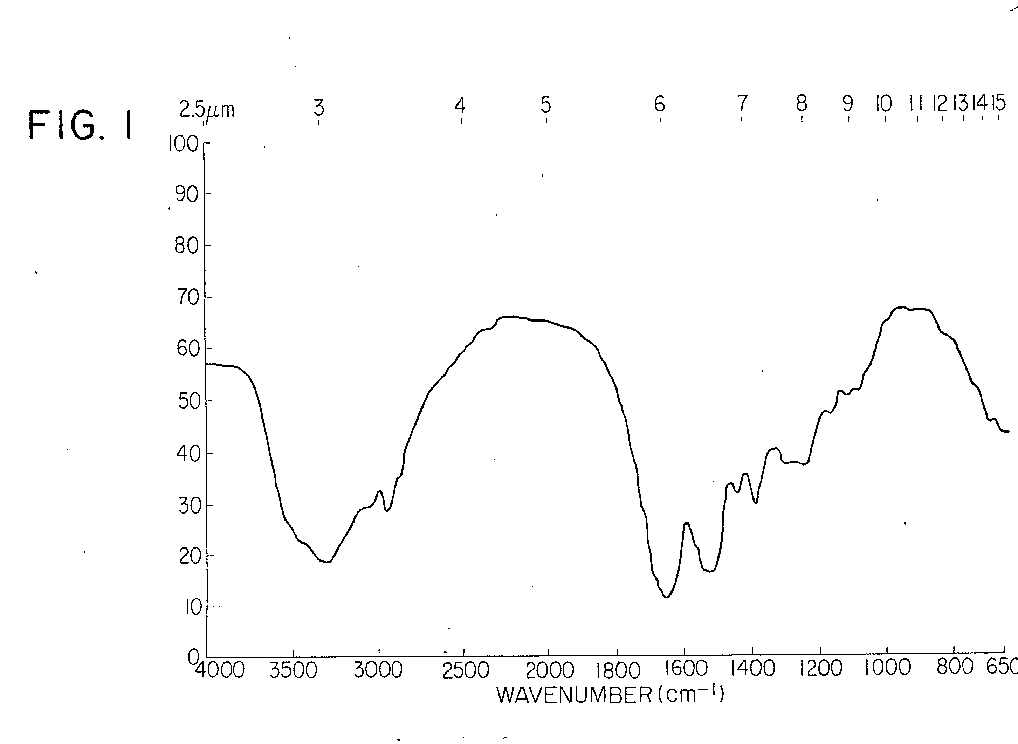

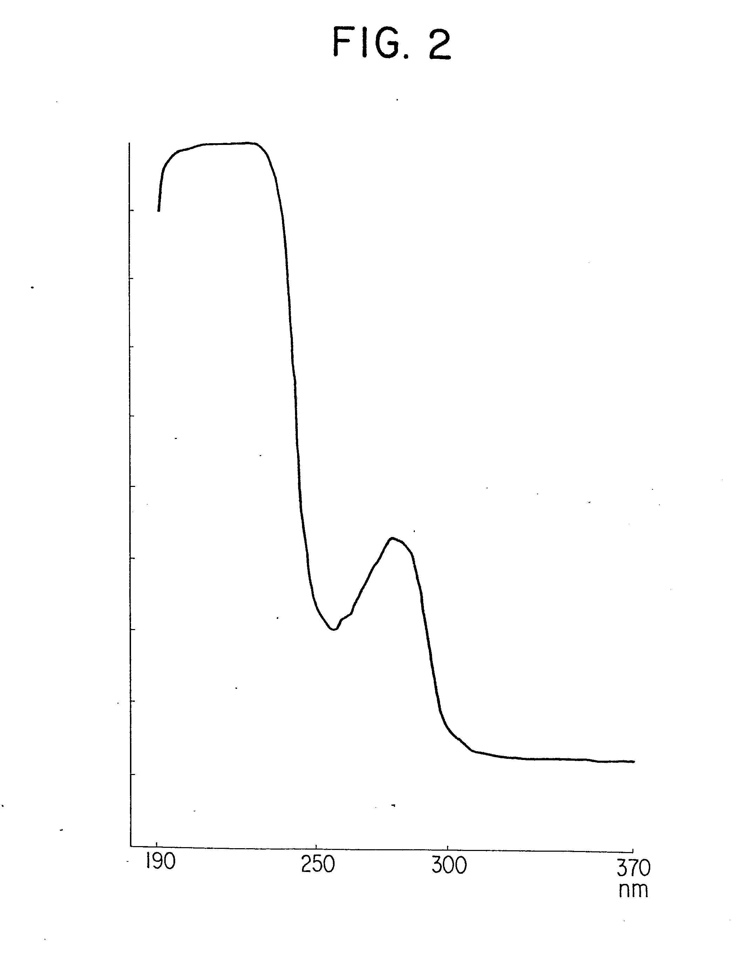

Fig. 1 illustrates the infrared absorption spectrum of
Santacuarator A according to the present invention. Figs. 2, 3
and 4 illustrate ultraviolet absorption spectrums at pH 3.0, pH
7.0 and pH 10.0 of Santacuarator A respectively. Fig. 5 illustrates the infrared absorption spectrum of Santacuarator B.

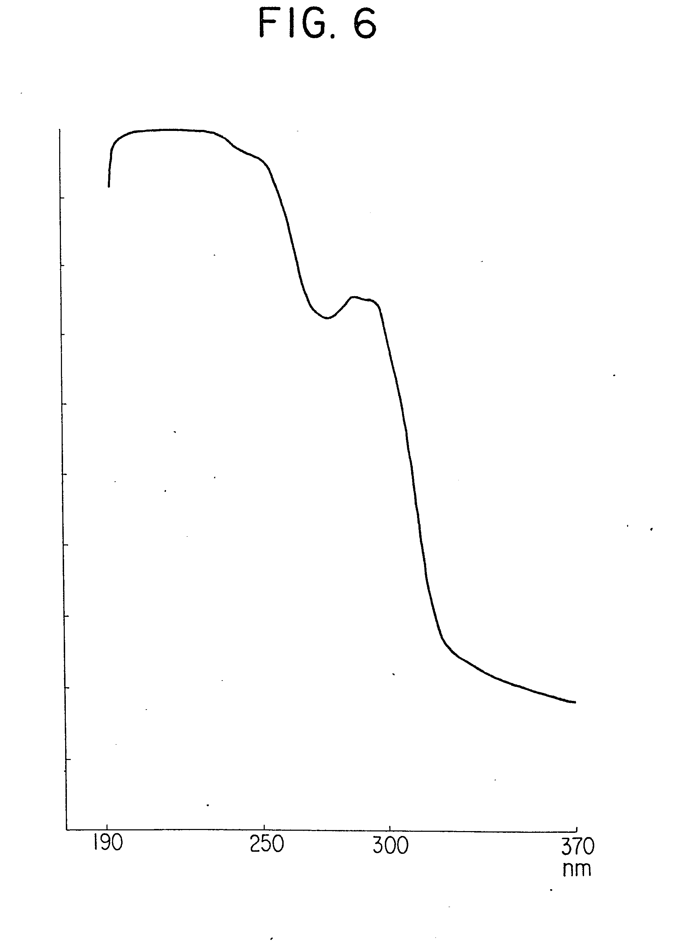

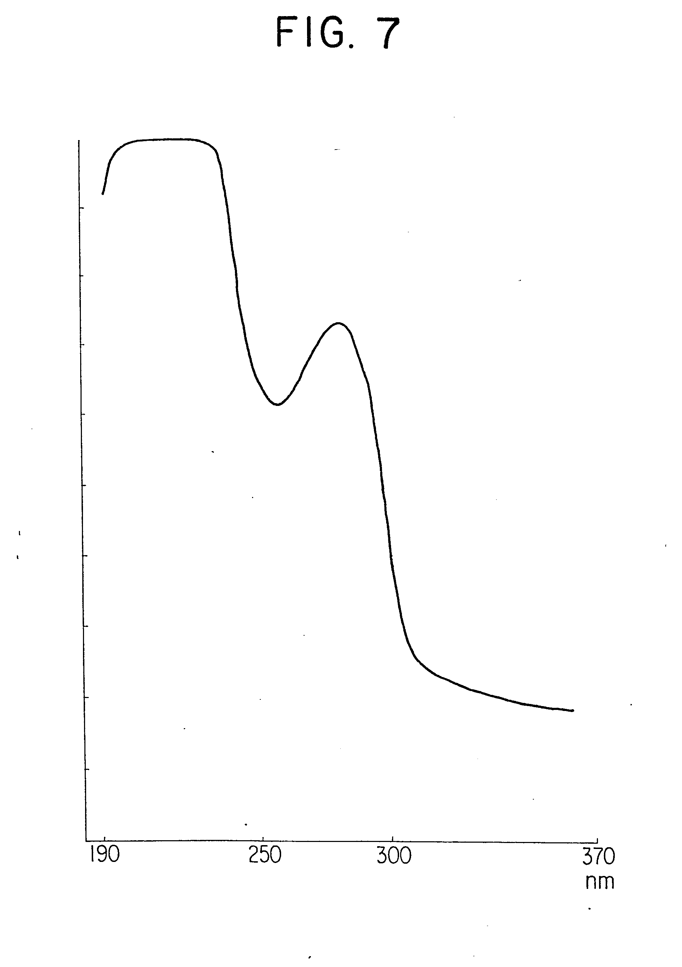

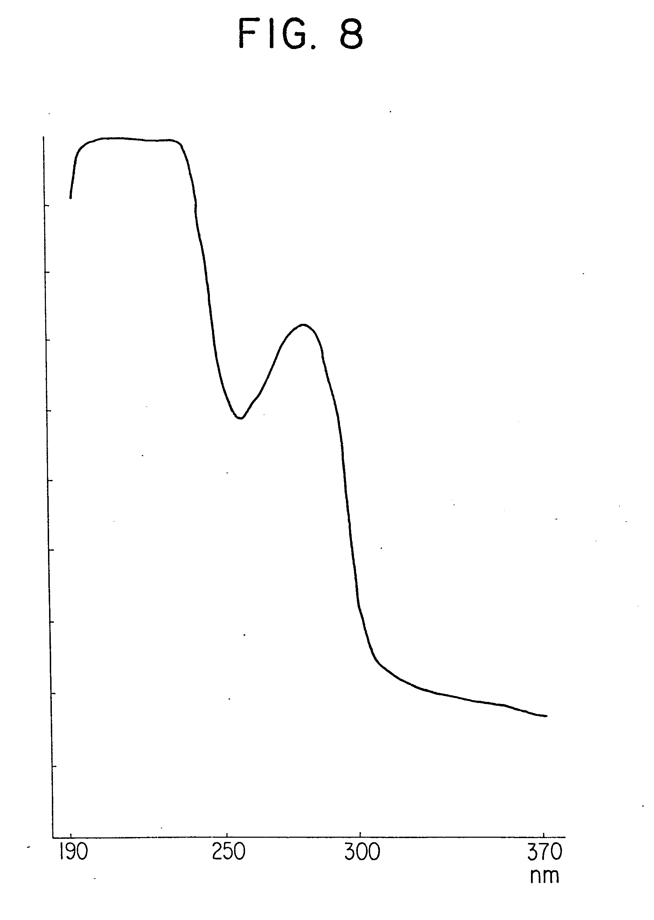

Figs. 6, 7 and 8 illustrate ultraviolet absorption spectrums at
pH 3.0, pH 7.0 and pH 10.0 of Santacuarator B respectively.
Fig. 9 illustrates the infrared absorption spectrum of Santacuarator C. Figs. 10, 11 and 12 illustrate ultraviolet absorption
spectrums at pH 3.0, pH 7.0 and pH 10.0 of Santacuarator C
respectively.

Figs. 13, 14 and 15 illustrate infrared absorption spectrums
of Daémocuarator A, B and C according to the present invention
respectively.

This novel protein Santacuarator according to the present ninhydrine, anthrone, Molish and periodic acid reactions; represents the infrared absorption spectrum [KBr pellet] shown in Fig. 1; and exhibits the maximum of ultraviolet absorption at 279 nm in pH 3 water, 278 nm in pH 7 water and 277 nm in pH 10.0 water respectively.

A further embodiment of the present invention consists in a protein named Santacuarator B fractionated from human or animal blood, serum or extract of removal organ and having a regulative action on proliferation of cancer cells, which is a white powder having a melting point of 220 - 225°C (decomposition); has a molecular weight of about 22,000 measured by 10% sodium dodecylsulfate electrophoresis; is soluble in water and insoluble in acetone, ethyl acetate, benzene, hexane and chloroform; has an aminoacid composition comprising aspartic acid 8.9%, threonine 6.6%, serine 6.4%, gultamic acid 10.9%, proline 4.8%, glycine 4.4%, alanine 7.1%, cysteine 12.3%, valine 5.8%, methionine 0.7%, isoleucine 2.4%, leucine 8.8%, tyrosine 2.8%, phenylalanine 5.0%, lysine 7.6%, histidine 2.5% and arginine 3.6%; is positive in ninhydrine reaction and negative in anthrone, Molish and periodic acid reactions; represents the infrared absorption spectrum [KBr pellet] shown in Fig. 5; and exhibits the maximums of ultraviolet absorption at 283 nm and 289 nm in pH 3 water, at 280 nm in pH 7 water and at 278 nm in pH 10.0 water respectively.

Still a further embodiment of the present invention consists in a protein named Santacuarator C fractionated from human or animal blood, serum or extract of removal organ and having a regulative action on proliferation of cancer cells, which is a white powder having a melting point of 235 - 240°C (decomposition); has a molecular weight of about 14,000 measured by 10% sodium dodecylsulfate electrophoresis; is soluble in water and insoluble

Example 1

10 ℓ calf serum was brought to 40% saturation by the addition of an aqueous ammonium sulfate solution to thereby obtain precipitate. The thus obtained precipitate was dissolved and this was adsorbed on 40 mℓ volume Sephadex G-200 at pH 7.3 and eluted with 60 mℓ Tris-HCℓ buffer (0.05M, pH 7.3) to obtain 10.5 g crude Santacuarator.

Next, this crude product was adsorbed on Diethylaminoethyl (DEAE) cellulose (produced by Wattman Co.), eluted by 40 mℓ Tris-HCℓ buffer (pH 7.4) having a gredient of ionic strength of from 0.02M to 0.05M and divided roughly into 5 fractions. The second fraction was dialyzed with 0.02M Tris buffer, then desalted, frozen and dried to obtain 49.7 mg crude product. This was further adsorbed on Diethylaminoethyl (DEAE) cellulose (produced by Wattman Co.) equilibrated with 0.02M Tris buffer (pH 7.4) and eluted by 30 mℓ Tris-HCℓ buffer (pH 7.4) having a gradient of ionic strength of from 0.02M to 0.05M and divided into 5 fractions. The second fraction was desalted, frozen and dried to obtain 7.1 mg white Santacuarator A.

This product was applied to electrophoresis to exhibit a single band.

The use of this product (0.03/mg/mℓ) exhibited 71.5% inhibitated effect on proliferation of Leukemia L-1210 cells in the tissue culture, and the use of this product (0.03/mg/mℓ) exhibited about 93% inhibitated activity.

Example 2

49 mg of the second fraction of those obtained firstly by chromatography using Diethylaminoethyl (DEAE) cellulose (produced by Wattman Co.) according to the same procedure as Example 1

was subjected to electrophoresis using acrylamide gel under the conditions: pH 8.0 and pH 10.0 to thereby fractionate the effective components into simple substances respectively. Each fraction was dialyzed, frozen and dried to thereby obtain 7 mg white powdery refined Santacuarator A respectively.

Example 3

1 ℓ human serum was treated according to the exactly same procedure as Example 1 to obtain 515 mcg Santacuarator A.

This was observed to effectively inhibit the proliferation of Leukemia L-1210 cells in the tissue culture and was confirmed to be the exactly same product as Santacuarator A separated from calf serum by means of electrophoresis using polyacrylamide gel under the conditions: pH 8.0, pH 9.5 and pH 10.0.

Example 4

1 Kg fresh calf liver was pulverized to extract 2 ℓ of 0.05M Tris buffer (pH 7.4). The liver extract was separated by centrifugation and 3 ℓ transparent extract was obtained.

This liver extract was treated by the extractly same procedure as Example 1, thereby obtaining 1.9 mg refined Santacuarator. This was observed to effectively inhibit the proliferation of Leukemia L-1210 cells in the tissue culture and was confirmed to be the exactly same substance as Santacuarator A separated from calf serum by means of electrophoresis using polyacrylamide gel under the conditions: pH 8.0, pH 9.5 and pH 10.0.

Example 5

10 ℓ calf serum was brought to 95% saturation by the addition of ammonium sulfate. The resulting precipitate was dissolved and this was adsorbed on 40 mℓ volume Sephadex G-50 at pH 7.3 and eluted with 50 mℓ Tris-HCℓ buffer (0.05M, pH 7.3) to thereby obtain 6.3 g crude Santacuarator.

Next, this crude product was adsorbed on Diethylaminoethyl (DEAE) cellulose (produced by Wattman Co.), eluted by 50 mℓ Tris-HCℓ buffer (pH 7.3) having a gradient of ionic strength of from 0.02M to 0.05M and divided roughly into 6 fractions. The first fraction was dialyzed desalted, frozen and dried to obtain 30 mg crude product.

This product was further adsorbed on Diethylaminoethyl (DEAE) cellulose (produced by Wattman Co.) equilibrated with 0.02M Tris buffer (pH 7.3) and eluted by 40 mℓ Tris-HCℓ buffer (pH 7.3) having a gradient of ionic strength of from 0.02M to 0.05M and divided into 6 fractions. The first fraction was dialyzed, desalted, frozen and dried to obtain 5 mg white Santacuarator B.

This product, when used in the amount of 500 mcg/mℓ, exhibited the activity to delay the angle in growth curve of Leukemia L-1210 cells in the tissue culture by 20 degrees, and exhibited a single band when applied to electrophoresis.

Example 6

1 ℓ human serum was treated by the exactly same procedure as Example 5 to obtain 362 mg Santacuarator B.

This product exhibited the same activity to check the angle in growth curve of Leukemia L-1210 cells in the tissue culture as exhibited in Example 5, and was confirmed to be the exactly same product as Santacuarator B separated from calf serum by means of electrophoresis using polyacrylamide gel under the conditions: pH 8.0, pH 9.5 and pH 10.0.

Example 7

1 Kg fresh calf liver was pulverized to extract 2 ℓ of 0.05M Tris buffer (pH 7.4). The liver extract was separated by centrifugation and 3 ℓ transparent extract was obtained. This liver extract was treated according to the exactly same procedure as Example 5, thereby obtaining 1.34 mg Santacuarator B.

This product was observed to exhibit the same activity to check the angle in growth curve of Leukemia L-1210 cells in the tissue culture as exhibited in Example 5, and was confirmed to be the exactly same one as Santacuarator B separated from calf serum by means of electrophoresis using polyacrylamide gel under the conditions: pH 8.0, pH 9.5 and pH 10.0.

Example 8

10 ℓ calf serum was saturated with ammonium sulfate. The resulting precipitate was dissolved and this was adsorbed on 40 mℓ volume Sephadex G-50 at pH 7.3 and eluted by 30 mℓ Tris-HCℓ buffer (pH 7.3, 0.05M), thereby obtaining 1.1 g crude Santacuarator.

In succession, this product was adsorbed on Diethylaminoethyl (DEAE) cellulose (produced by Wattman Co.), eluted by 40 mℓ Tris-HCℓ buffer (pH 7.3) having a gradient of ionic strength of from 0.02 to 0.05M, and divided roughly into 3 fractions. The second fraction was dialyzed, desalted, frozen and dried to obtain 11.6 mg crude product. This was further adsorbed on Diethylaminoethyl (DEAE) cellulose (produced by Wattman Co.), eluted by 40 mℓ Tris-HCℓ buffer (pH 7.3) having a gradient of ionic strength of from 0.02 to 0.05M, and divided roughly into 3 fractions. The second fraction was dialyzed, desalted, frozen and dried to obtain 11.6 mg crude product. This product was further adsorbed on Diethylaminoethyl (DEAE) cellulose (produced by Wattman Co.) equilibrated

BAD ORIGINAL

with 0.02M Tris buffer (pH 7.3), eluted by Tris-HCl buffer (pH 7.3) having a gradient of ionic strength of from 0.02 to 0.05M, and divided into 6 fractions. The fourth fraction was dialyzed, desalted, frozen and dried to obtain 2.9 mg white Santacuarator C.

This product exhibited a single band when applied to electrophoresis, and, when used in the amount of 500 mcg/mℓ, exhibited the activity to delay the angle in growth curve of Leukemia L-1210 cells (as tissue caltured) by 10 degrees.

Example 9

1 ℓ human serum was treated according to the exactly same procedure as Example 8 to obtain 148 mcg Santacuarator C.

This product exhibited the same activity to check the angle in growth curve of Leukemia L-1210 cells as shown in Example 8, and confirmed to be the exactly same one as Santacuarator C separated from calf serum by means of electrophoresis using polyacrylamide gel under the conditions: pH 8.0, pH 9.5 and pH 10.0.

Example 10

1 Kg fresh calf liver was pulverized to extract 2 ℓ of 0.05M Tris buffer (pH 7.4). This liver extract was separated by centrifugation and about 3 ℓ of transparent extract was obtained. This liver extract was treated according to the exactly same procedure as shown in Example 8, thereby obtaining 717 mcg refined Santacuarator C.

This product was observed to exhibit the same activity to check the angle in growth curve of tissue cultured Leukemia L-1210 cells as shown in Example 8, and was confirmed to be the same one as Santacuarator C separated from calf serum by means of electrophoresis using polyacrylamide gel under the conditions: pH 8.0,

Example 11

By using 500 mcg/ml Santacuarator A extracted from human and calf serum or calf liver according to Example 1, Example 2, Example 3 and Example 4, their regulative effects on proliferation of cancer cells were measured.

The measured regulative effects on proliferation of cancer cells were 30.6% against Leukemia L5178Y cell, 19.4% against Sarcoma S-180 cell, and 31.6% against Ehrlich carcinoma cell, but no inhibitated action on proliferation of cancer cells was exhibited against the primary tissue cultured cell.

Example 12

Animal tests were carried out using Santacuarator A obtained according to Example 1.

Tested animal groups were each comprised of five $BDF_1$ mice (weighing 18.5 g).

In the first group, 1.1 mg/head of Santacuarator was injected in the abdominal cavity and simultaneously Leukemia L-1210 cell ($1 \times 10^5$) was injected by i.p. In the second group, on the other hand, the same experiment as done in the first group was carried out and thereafter 0.08 mg/head of Santacuarator A was injected by i.p. on the fourth day after the first injection. The average life of control mice was 10 days, but the whole mice in the first and second groups lived up to 60 days after treatment and exhibited the same weight increasing curve as non-treated $BDF_1$ mice did. The tested mice were subjected to decapitation and ventrotomy on the 60th day, but the presence of abdominal dropsy could not be observed from any mouse.

0085254

4 mg/head of Santacuarator A was injected by i.p. into a group of mice, but no example of death could be found for 60 days.

Example 13

By using each Santacuarator B (500 mcg/mℓ) extracted from human and calf serum or calf liver according to Examples 5, 6 and 7, tests were carried to show that they exhibited the activities to delay the angle in cell growth curve by 14 degrees against tissue cultured Leukemia L5178Y cell, by 13 degrees against tissue cultured Sarcoma S-180 cell and by 4 degrees against tissue cultured Ehrlich carcinoma cell. However, any regulative action was not exhibited on the proliferation of the primary tissue cultured cell derived from rat liver. And, various cancer cells being tested were all confirmed to live by dyening test.

Example 14

By using each Santacuarator C (500 mcg/mℓ) extracted from human and calf serum or calf liver according to Examples 8, 9 and 10, tests were carried out to show that they exhibited the activities to delay the angle in cell growth curve by 1 degree against tissue cultured Leukemia L5187Y cell, by 20 degrees against tissue cultured Sarcoma S-180 cell and by 1 degree against tissue cultured Ehrlich carcinoma cell. However, any regulative action was not exhibited on the proliferation of the primary tissue cultured cell derived from rat liver. And, various cancer cells being tested were all confirmed to live by dyening test.

In this connection, the same animal tests as Example 12 were carried out with reference to Santacuarator B and C obtained according to Examples 5 to 10 to confirm that every one inhibited the proliferation of Leukemia L-1210 cell effectively as done in Example 12 and did not exert any marked virulence on animals.

Example 15

200 mℓ calf serum was extracted four times with 50 mℓ of hexane (a solvent) and thereafter said solvent was removed, thereby obtaining 21.13 mg crude Daemocuarator product. 4.4 mg/head of the obtained crude Daemocuarator product was injected by i.p. into five $BDF_1$ mice (weighing 18.5 g) transplanted with $1 \times 10^5$ Leukemia L-1210 cell. Consequently, it was found that the life time of said $BDF_1$ mice was reduced to 8.3 days in contrast with the fact that the control mouse which had been given the cancer cell alone survived for 10.7 days.

On the other hand, 1 mℓ/head of the serum obtained by removing under reduced pressure the hexane contained in the residual serum after Daemocuarator has been extracted was injected by i.p. into five $BDF_1$ mice (weighing 18.5 g) transplanted with $1 \times 10^5$ Leukemia L-1210 cell to find that their average life time was prolonged to 16.0 days in contrast with the fact that the life time of the control mouse was 10.3 days.

Example 16

200 mℓ of calf blood was extracted four times with 50 mℓ benzene, thereafter said solvent was removed, thereby obtaining 20.43 mg crude Daemocuarator product. 4.4 mg/head of the thus obtained crude Daemocuarator product was injected by i.p. into five $BDF_1$ mice (weighing 18.5 g) transplanted with $1 \times 10^5$ Leukemia L-1210 cell. Consequently, the life time of said $BDF_1$ mouse was reduced to 9.3 days on average in contrast with the fact that the control mouse which had been given the cancer cell alone had the life time of 10.7 days.

On the other hand, 1 mℓ/head of the blood obtained by removing under reduced pressure the benzene contained in the residual blood after Daemocuarator had been extracted was injected by i.p. into five $BDF_1$ mice (weighing 18.5 g) transplanted with $1 \times 10^5$ Leukemia L-1210 cell to find that their average life time was prolonged to 15.7 days in contrast with the fact that the life time of the control mouse was 10.3 days.

Example 17

200 mℓ of calf serum was extracted four times with 50 mℓ butanol, and likewise 28.74 mg crude Daemocuarator product was obtained. 4.4 mg/head of the thus obtained crude Daemocuarator product was injected by i.p. into five $BDF_1$ mice (weighing 18.5 g) transplanted with $1 \times 10^5$ Leukemia L-1210 cell to find that the average life time of $BDF_1$ mice was reduced to 10.0 days in contrast with the fact that the life time of the control mouse which had been given the cancer cell alone was 10.7 days.

On the other hand, the butanol contained in the residual serum after Daemocuarator had been extracted was extracted four times with 50 mℓ of benzene and thereafter the residual butanol was removed at reduced pressure completely. 1 mℓ/head of the thus obtained serum was injected by i.p. into five $BDF_1$ mice (weighing 18.5 g) transplanted with $1 \times 10^5$ Leukemia L-1210 cell to find that their average life time was prolonged to 15.3 days in contrast with the fact that the life time of the control mouse was 10.3 days.

Example 18

200 mℓ of calf blood was extracted four times 50 mℓ methylethyl ketone, and likewise 29.91 mg crude Daemocuarator product was obtained. 4.4 mg/head of the thus obtained crude Daemocuarator product was injected by i.p. into five $BDF_1$ mice (weighing 18.5 g) transplanted with $1 \times 10^5$ Leukemia L-1210 cell to find that the average life time of said $BDF_1$ mice was reduced to 7.3 days in contrast with the fact that the life time of the control mouse which had been given the cancer cell alone was 10.7 days.

On the other hand, the methylethyl ketone contained in the residual blood after Daemocuarator had been extracted was extracted four times with 50 mℓ of benzene and thereafter the organic solvent added was removed completely at reduced pressure. 1 mℓ/head of the thus obtained blood was injected by i.p. into five $BDF_1$ mice (weighing 18.5 g) transplanted with $1 \times 10^5$ Leukemia L-1210 cell to find that their average life time was prolonged to 20.7 days in contrast with the fact that the life time of the control mouse was 10.3 days.

Example 19

200 mℓ of human serum was extracted four times with 50 mℓ hexane and thereafter said solvent was removed, thereby obtaining 19.87 mg crude Daemocuarator product was obtained. 4 mg/head of the obtained crude Daemocuarator product was injected by i.p. into five $BDF_1$ mice (weighing 18.5 g) transplanted with $1 \times 10^5$ Leukemia L-1210 cell to find that the life time of said $BDF_1$ mouse was reduced to 8.0 days in contrast with the fact that the control mouse which had been given the cancer cell alone was 10.0 days.

On the other hand, 2 mℓ/head of the serum obtained by removing the hexane contained in the residual serum after Daemocuarator had been extracted at reduced pressure was injected by i.p. into five $BDF_1$ mice (weighing 18.5 g) transplanted with $1 \times 10^5$ Leukemia L-1210 cell to find that their average life time was prolonged to 18.0 days in contrast with the fact that the life time of the control mouse was 10.0 days.

Example 20

200 mℓ of human blood was extracted four times with 50 mℓ benzene, and likewise 22.31 mg crude Daemocuarator product was obtained. 4 mg/head of the thus obtained crude Daemocuarator product was injected by i.p. into five $BDF_1$ mice (weighing 18.5 g) transplanted with $1 \times 10^5$ Leukemia L-1210 cell to find that the life time of said $BDF_1$ mouse was reduced to 7.6 days in contrast with the fact that the life time of the control mouse which had been given the cancer cell alone was 10.0 days.

On the other hand, 2 mℓ/head of the blood obtained by removing the benzene contained in the residual blood after Daemocuarator had been extracted at reduced pressure was injected by i.p. into five $BDF_1$ mice (weighing 18.5 g) transplanted with $1 \times 10^5$ Leukemia L-1210 cell to find that their average life time was prolonged to 17.3 days in contrast with the fact that the life time of the control mouse was 10.0 days.

Example 21

200 mℓ of human serum was extracted four times with 50 mℓ butanol and said solvent was removed to thereby obtain 29.81 mg of crude Daemocuarator product. 4 mg/head of the thus obtained crude Daemocuarator product was injected by i.p. into five $BDF_1$ mice (weighing 18.5 g) transplanted with $1 \times 10^5$ Leukemia L-1210 cell to find that the life time of said $BDF_1$ mouse was reduced to 8.3 days on average in contrast with the fact that the life time of the control mouse which had been given the cancer cell alone was 10.0 days.

On the other hand, the butanol contained in the residual serum after Daemocuarator had been extracted was extracted four times with 50 m$\ell$ benzene, and the remaining butanol was removed completely at reduced pressure 2 m$\ell$/head of the thus obtained serum was injected by i.p. into five BDF$_1$ mice (weighing 18.5 g) transplanted with 1 x 10$^5$ Leukemia L-1210 cell to find that their average life time was prolonged to 16.6 days in contrast with the fact that the life time of the control mouse was 10.0 days.

Example 22

200 m$\ell$ of human blood was extracted four times with 50 m$\ell$ methylethyl ketone and likewise 27.71 mg crude Daemocuarator product was obtained. 4 mg/head of the thus obtained crude Daemocuarator product was injected by i.p. into five BDF$_1$ mice (weighing 18.5 g) transplanted with 1 x 10$^5$ Leukemia L-1210 cell to find that the average life time of said BDF$_1$ mice was reduced to 7.3 days in contrast with the fact that the life time of the control mouse which had been given the cancer cell alone was 10.0 days.

On the other hand, the methylethyl ketone contained in the residual blood after Daemocuarator had been extracted was extracted four times with 50 m$\ell$ benzene and thereafter the organic solvent added was further removed completely at reduced pressure. 2 m$\ell$/head of the thus obtained blood was injected by i.p. into five BDF$_1$ mice (weighing 18.5 g) transplanted with 1 x 10$^5$ Leukemia L-1210 cell to find that their average life time was prolonged to 21.0 days in contrast with the fact that the life time of the control mouse was 10.0 days.

Example 23

100 g of calf liver was pulverized and extracted with 50 ml of 0.05M Tris buffer (pH 7.0). The resulting liver extract was separated by centrifugation to obtain 100 ml transparent extract. This extract was treated according to the exactly same procedure as Example 1, thereby obtaining 28.3 mg crude Daemocuarator product. 8 mg/head of the thus obtained crude Daemocuarator product was injected by i.p. into five $BDF_1$ mice (weighing 18.5 g) transplanted with $1 \times 10^5$ Leukemia L-1210 cell to find that the life time of said $BDF_1$ mouse was reduced to 9.3 days in contrast with the fact that the control mouse which had been given the cancer cell alone survived for 10.7 days.

On the other hand, 5 ml/head of the extract obtained by removing the organic solvent contained in the residual extract after Daemocuarator had been extracted at reduced pressure was injected by i.p. into five $BDF_1$ mice (weighing 18.5 g) transplanted with $1 \times 10^5$ Leukemia L-1210 cell to find that the average life time of said $BDF_1$ mice was prolonged to 18.3 days in contrast with the fact that the life time of the control mouse which had been given the cancer cell alone was 10.0 days.

CLAIMS

1.    An extract obtained from animal blood, serum or organ using an organic solvent and/or an adsorbent, in which the extract contains less than the blood, serum or organ of a substance antagonistic to a factor able to regulate the proliferation of cancer cells.

2.    A glycoprotein named Santacuarator A fractionated from animal blood, serum or          organ  and which can have regulative action on proliferation of cancer cells, which is a white powder having a melting point of 240 - 250°C (decomposition); has a molecular weight of about 74000 measured by electrophoresis using 10% sodium dodecylsulfate; is soluble in water and insoluble in butanol, acetone, ethyl, acetate, chloroform, benzene and hexane; has an aminoacid composition comprising aspartic acid 9.7%, threonine 9.3%, serine 12.3%, glutamic acid 10.0%, proline 5.8%, glycine 7.7%, alanine 5.8%, valine 8.8%, methionine 0.8%, isoleucine 3.2%, leucine 7.5%, tyrosine 3.5%, phenylalanine 3.5%, lysine 6.2%, histidine 1.8% and arginine 4.0%; is positive in the ninhydrin , anthrone, Molish and periodic acid reactions; exhibits   the infrared absorption spectrum [KBr pellet] shown in Fig. 1; and exhibits    maxima  of ultraviolet absorption at 279 nm at pH 3     ·., 278 nm at pH 7      'and 277 nm at pH 10.0.


3.  . A protein named Santacuarator B fractionated from animal blood, serum or          organ and which can have    regulative action on proliferation of cancer cells, which is a white powder having a melting point of 220 - 225°C (decomposition); has a molecular weight of about 22000 measured by electrophoresis using 10% sodium dodecylsulfate; is soluble in water and insoluble in acetone, ethyl acetate, benzene, hexane and chloroform; has an aminoacid composition comprising aspartic acid 8.9%, threonine 6.6%, serine 6.4%, glutamic acid 10.9%, proline 4.8%, glycine 4.4%, alanine 7.1%, cysteine 12.3%, valine 5.8%, methionine 0.7%, isoleucine 2.4%, leucine 8.8%, tyrosine 2.8%, phenylalanine 5.0%,

lysine 7.6%, histidine 2.5% and arginine 3.6%; is positive in the ninhydrin reaction and negative in anthrone, Molish and periodic acid reactions; exhibits the infrared absorption spectrum [KBr pellet] shown in Fig. 5; and exhibits maxima of ultraviolet absorption at 283 nm and 289 nm at pH 3, 280 nm at pH 7 and 278 nm at pH 10.0.

4. A protein named Santacuarator C fractionated from animal blood, serum or organ and which can have regulative action on proliferation of cancer cells, which is a white powder having a melting point of 235 - 240°C (decomposition); has a molecular weight of about 14000 measured by electrophoresis using 10% sodium dodecylsulfate; is soluble in water and insoluble in acetone, ethyl acetate, benzene, hexane and chloroform; has an aminoacid composition comprising aspartic acid 10.5%, threonine 7.1%, serine 6.2%, glutamic acid 12.5%, proline 5.2%, glycine 4.7%, alanine 8.0%, cysteine 1.7%, valine 6.1%, methionine 0.7%, isoleucine 2.7%, leucine 10.5%, tyrosine 3.1%, phenylalanine 5.7%, lysine 8.5%, histidine 2.8% and arginine 4.1%; is positive in the ninhydrin reaction and negative in anthrone, Molish and periodic acid reactions; exhibits the infrared absorption spectrum [KBr pellet] shown in Fig. 9; and exhibits a shoulder of ultraviolet absorption at 280 nm at pH 3 and maxima of ultraviolet absorption at 290 nm at pH 7 and at 277 nm at pH 10.0.

5.    An extract according to claim 1, which is a complex comprising Santacuarators A, B and C, obtained by precipitating the blood, serum or organ by salting out or by the addition of an organic solvent.

6.    An extract according to any preceding claim, obtained from mammalian blood, serum or organ.

7.    An extract according to any preceding claim, wherein the organ is the liver or kidney.

8.    An extract according to claim 6, obtained from human or calf blood or serum or calf liver.

9.    An extract according to any preceding claim, obtained by using an organic solvent selected from aliphatic hydrocarbons such as benzene or hexane, chlorinated hydrocarbons such as chloroform, alcohols such as butanol, ketones such as methyl ethyl ketone, esters such as ethyl acetate, and nitriles.

10.    A Daemocuarator as herein defined.

FIG. 1

# FIG. 2

# FIG. 3

# FIG. 4

FIG. 5

# FIG. 6

190          250          300          370
                                        nm

# FIG. 7

0085254

# FIG. 8

190        250        300        370
                                 nm

FIG. 9

9/15

0085254

# FIG. 10

0085254

# FIG. 11

190          250          300          370
                                        nm

# FIG. 12

190    250    300    370
nm

FIG. 13

13/15

0085254

FIG. 14

WAVENUMBER (cm⁻¹)

14/15

0085254

FIG. 15

15/15

0085254

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | EP-A-0 035 102   (RICHTER GEDEON VEGYESZETI GYAR) <br> * Claim 1 * | 1 | A 61 K   35/14 <br> A 61 K   37/02 <br> C 07 G    7/00 |
| A,P | EP-A-0 049 611   (HOOPER TRADING) <br> * Abstract * | 1 | |
| A | DE-A-2 806 146   (RESEARCH CORP.) <br> * Claim 1 * | 1 | |
| A | US-A-4 261 976   (K.J. ISSELBACHER et al.) <br> * Claim 1 * | 1 | |
| A,P | US-A-4 309 418   (S. GREEN) <br> * Claim 1 * | 1 | |

TECHNICAL FIELDS
SEARCHED (Int. Cl. ³)

A 61 K   35/14
A 61 K   35/16
A 61 K   37/02
C 07 G    7/00

The present search report has been drawn up for all claims

| Place of search <br> BERLIN | Date of completion of the search <br> 18-03-1983 | Examiner <br> KNAACK M |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03.82